# EUROPEAN PATENT SPECIFICATION

(11) **EP 4 337 291 B1**
(45) Date of publication and mention of the grant of the patent: **13.08.2025**
(21) Application number: 23816311.7
(22) Date of filing: 26.05.2023
(51) Int. Cl.: A61M 21/00, A61N 1/36, A61M 21/02, A61N 1/08, A61N 1/04

(54) **BRAIN STIMULATION DEVICE AND BRAIN STIMULATION SYSTEM INCLUDING THE SAME**
GEHIRNSTIMULATIONSVORRICHTUNG UND GEHIRNSTIMULATIONSSYSTEM DAMIT
DISPOSITIF DE STIMULATION CÉRÉBRALE ET SYSTÈME DE STIMULATION CÉRÉBRALE LE COMPRENANT

(30) Priority: 02.06.2022 KR 20220067693; 09.08.2022 KR 20220099429
(43) Date of publication of application: 20.03.2024
(73) Proprietor: KT & G Corporation, Daejeon 34337 (KR)
(72) Inventor: LEE, Won Kyeong, Guri-si, Gyeonggi-do 11920 (KR); KIM, Min Kyu, Seoul 08211 (KR); SUNWOO, Paul Joon, Seoul 06676 (KR)
(74) Representative: Ter Meer Steinmeister & Partner
(86) International application number: PCT/KR2023/007255
(87) International publication number: WO 2023/234650

(56) References cited:
- CN-A- 109 645 992
- JP-A- 2009 537 226
- KR-A- 20180 127 460
- US-A1- 2005 280 544
- US-A1- 2011 288 610
- US-A1- 2015 335 877
- US-A1- 2015 335 877
- US-A1- 2020 093 185
- US-A1- 2021 052 883

## Description

### Technical Field

The disclosure relates to a brain stimulation device and a brain stimulation system including the same, and more particularly, to a brain stimulation device capable of providing a user with brain stimulation corresponding to a smoking behavior, and a brain stimulation system including the same.

### Background Art

As studies such as molecular biology, evolutionary biology, electrophysiology, and bioinformatics develop, research on brain science, which analyzes the complex functions and structures of the brain and studies methods for activating various functions of the brain, has gradually increased.

As research on brain science progresses, the development of brain stimulation devices aimed at treating diseases by applying electrical stimulation to nerves has increased. After being attached to users' bodies (e.g., foreheads), brain stimulation devices may apply electrical stimulation to the users' nerves by flowing microcurrents through their bodies.

Brain stimulation devices as described above have been mainly used for treating diseases, such as depression and dementia, or for relieving stress of users. However, as the development of brain stimulation devices progresses, attempts to use nerve stimulation devices in various fields as well as for purposes of disease treatment or stress relief have gradually increased.

US 2011/288610 A1 relates to a device for transcranial electric current stimulation, comprising the following components: -electrodes with fastening means for exactly positioning on the skin of the head and electrical connecting lines and -a transportable, miniaturized stimulation generator comprising a current generator, a controller, a user interface, an electrical energy storage device and a monitoring and safety module with a separate electrical energy storage device.

### Disclosure of Invention

### Technical Problem

When electrical stimulation corresponding to brain waves changed due to smoking is applied to a user by measuring and analyzing the brain waves of the user before and after smoking, the user may feel brain stimulation corresponding to a smoking behavior even without a separate smoking behavior.

Provided are a brain stimulation device capable of providing a smoking sensation to a user via electrical stimulation even without a smoking behavior, and a brain stimulation system including the same to extend the fields of use of the brain stimulation device.

The technical problems of the disclosure are not limited to the above-described description, and other technical problems may be clearly understood by one of ordinary skill in the art from the embodiments to be described hereinafter.

### Solution to Problem

The invention provides a brain stimulation device according to claim 1 and a brain stimulation system according to claim 6. Embodiments of the invention are defined in the dependent claims. For better understanding of the invention the present disclosure provides also further examples of brain stimulation devices and systems.

According to an aspect of the disclosure, a brain stimulation device includes a housing having at least one area attached to a body of a user, and including an accommodation space having an article accommodated therein, a battery arranged inside the housing, an output unit configured to apply a current to the body of the user on the basis of power supplied from the battery, and a processor electrically connected to the battery and the output unit, wherein the processor is configured to detect a type of the article accommodated in the accommodation space, and on the basis of the detected type of the article, apply a current to the body of the user via the output unit so that brain stimulation is applied to the user.

According to another aspect of the disclosure, a brain stimulation system includes a brain stimulation device having at least one area attached to a body of a user, and a control device operatively connected to the brain stimulation device, wherein the brain stimulation device includes a first housing having at least one area attached to the body of the user, a battery arranged inside the first housing, an output unit configured to apply a current to the body of the user on the basis of power supplied from the battery, and a first processor electrically connected to the battery and the output unit, and the control device includes a second housing including an accommodation space having an article accommodated therein, and a second processor configured to detect a type of the article accommodated in the accommodation space, wherein the first processor is configured to receive, from the second processor, a signal including data corresponding to the detected type of the article, and on the basis of the received signal, apply a current to the body of the user via the output unit so that brain stimulation is applied to the user.

### Advantageous Effects of Invention

A brain stimulation device and a brain stimulation system including the same, according to various embodiments, may provide brain stimulation corresponding to a smoking behavior via electrical stimulation even when a user does not conduct a smoking behavior.

In addition, the brain stimulation device and the brain stimulation system including the same, according to various embodiments, may provide a user with brain stimulation corresponding to a smoking behavior or relieve stress or tension of the user by providing various forms of electrical stimulation to the user.

Effects of the disclosure are not limited to the above effects, and effects that are not mentioned could be clearly understood by one of ordinary skill in the art from the present specification and the attached drawings.

### Brief Description of Drawings

FIG. 1 is a perspective view of a brain stimulation device according to an embodiment.
FIG. 2 is a view illustrating a process of applying a current to the body of a user.
FIG. 3 is a cross-sectional view illustrating an electrical connection relationship between the brain stimulation device of FIG. 1 and an article accommodated in the brain stimulation device.
FIG. 4 is a flowchart illustrating operations of a brain stimulation device applying a current on the basis of the type of an article, according to an embodiment.
FIG. 5 is a graph illustrating an example of a change in current applied to the body of a user when a first article is inserted into a brain stimulation device.
FIG. 6 is a graph illustrating an example of a change in current applied to the body of a user when a second article is inserted into a brain stimulation device.
FIG. 7 is a flowchart illustrating operations of a brain stimulation device controlling a current applied to the body of a user on the basis of the number of times a current is applied, according to an embodiment.
FIG. 8 is a perspective view illustrating a brain stimulation device and an external electronic device, according to an embodiment.
FIG. 9 is a perspective view illustrating a brain stimulation system according to an embodiment.
FIG. 10 is a diagram illustrating a process of applying, by the brain stimulation system of FIG. 9, a current to the body of a user.
FIG. 11 is a flowchart illustrating operations of a brain stimulation system applying a current on the basis of the type of an article, according to an embodiment.

### Mode for the Invention

Regarding the terms in the various embodiments, the general terms which are currently and widely used are selected in consideration of functions of structural elements in the various embodiments of the disclosure. However, meanings of the terms can be changed according to intention, a judicial precedence, the appearance of a new technology, and the like. In addition, in certain cases, terms which can be arbitrarily selected by the applicant in particular cases. In such a case, the meaning of the terms will be described in detail at the corresponding portion in the description of the disclosure. Therefore, the terms used in the various embodiments of the disclosure should be defined based on the meanings of the terms and the descriptions provided herein.

In addition, unless explicitly described to the contrary, the word "comprise" and variations such as "comprises" or "comprising" will be understood to imply the inclusion of stated elements but not the exclusion of any other elements. In addition, the terms "-er", "-or", and "module" described in the specification mean units for processing at least one function and operation and can be implemented by hardware components or software components and combinations thereof.

Hereinafter, the disclosure will now be described more fully with reference to the accompanying drawings, in which exemplary embodiments of the disclosure are shown such that one of ordinary skill in the art may easily work the disclosure. The disclosure may be implemented in various different forms, and is not limited to the embodiments described herein.

Hereinafter, embodiments will be described in detail with reference to the drawings.

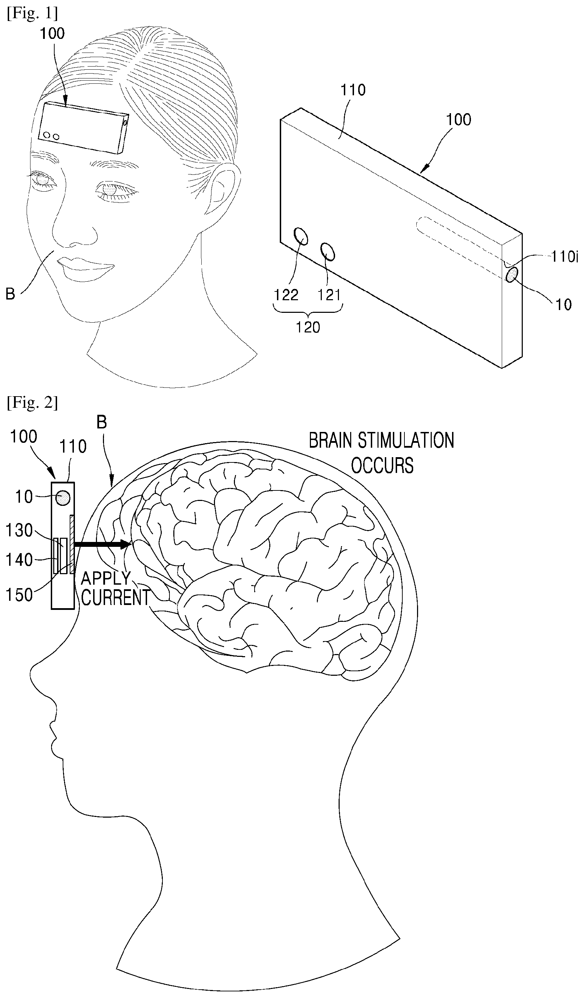

FIG. 1 is a perspective view of a brain stimulation device according to an embodiment.

Referring to FIG. 1, a brain stimulation device 100 according to an embodiment may have at least one area attached to a body B of a user, and may provide the user with brain stimulation corresponding to a smoking behavior by applying a current to the body B of the user.

In the disclosure, "brain stimulation corresponding to a smoking behavior" may refer to stimulation that is substantially the same as stimulation applied to a brain by a smoking behavior of a user. The user may feel the same sensory or psychological stability as a smoking behavior via brain stimulation corresponding to the smoking behavior. In addition, the user may relieve stress or tension via the brain stimulation corresponding to the smoking behavior. Here, a brain stimulation device of the disclosure may be referred to as a brain stimulation device for smoking, but is not limited thereto.

According to an embodiment, the brain stimulation device 100 may include a housing 110 having at least one area attached to the body B of the user and at least one button unit 120 for receiving an input of the user.

The housing 110 may form the overall appearance of the brain stimulation device 100, and may include an accommodation space 110i for accommodating an article 10. At least a partial area of the article 10 may be inserted or accommodated in the accommodation space 110i, and the brain stimulation device 100 may detect a type of the article 10 inserted into the accommodation space 110i and control brain stimulation applied to the user on the basis of the detected type of the article 10. A detailed description thereof will be given below.

In an example, the housing 110 may be formed to have a rectangular patch shape as a whole, but the shape of housing 110 is not limited thereto. In an example, the housing 110 may be formed to have an elliptical patch shape, or may be formed to have a polygonal patch shape.

In addition, as illustrated in FIG. 1, the housing 110 may be attached to a forehead of the user, but a part of the body B of the user to which the housing 110 is attached is not limited to the illustrated embodiment. In an example, housing 110 may also be attached to a ball or neck of the user.

The at least one button unit 120 may be arranged on an outer circumferential surface of the housing 110 to receive a user input. A processor (not shown) of the brain stimulation device 100 may control an operation of the brain stimulation device 100 on the basis of an input of the user to the at least one button unit 120.

According to an embodiment, the at least one button unit 120 may include a first button unit 121 for controlling a power state of the brain stimulation device 100 and a second button unit 122 for controlling an intensity of brain stimulation applied to the user. However, the number of the at least one button unit 120 is not limited to the embodiment described above, and the number of button units may increase or decrease according to embodiments.

In an example, when an input of the user to the first button unit 121 is received, the processor of the brain stimulation device 100 may switch the power state of the brain stimulation device 100 from an on state to an off state, or conversely, from the off state to the on state.

In an example, when an input of the user to the second button unit 122 is received, the processor of the brain stimulation device 100 may control an intensity of brain stimulation applied to the user by adjusting an intensity of a current applied to the user.

For example, on the basis of the input of the user to the second button unit 122, the processor may adjust the intensity of the current applied to the user so that strong brain stimulation is applied to the user or brain stimulation is applied quickly. As another example, on the basis of the input of the user to the second button unit 122, the processor may adjust the intensity of the current applied to the user so that relatively weak brain stimulation is applied to the user or brain stimulation is applied slowly.

In addition, on the basis of the input of the user to the second button unit 122, the processor may also adjust a frequency of the current applied to the user to adjust an intensity of brain stimulation applied to the user or a time for which the brain stimulation is applied.

Hereinafter, components of the brain stimulation device 100 for applying a current to a body of a user will be described in detail with reference to FIG. 2.

FIG. 2 is a view illustrating a process of applying, by a brain stimulation device as illustrated in FIG. 1, a current to the body of a user.

Referring to FIG. 2, a brain stimulation device 100 according to an embodiment may include a housing 110, a processor 130, a battery 140, and an output unit 150. At least one of components of the brain stimulation device 100 according to an embodiment may be the same as or similar to at least one of the components of the brain stimulation device 100 of FIG. 1, and the same description thereof will be omitted below.

The housing 110 may provide an accommodation space (e.g., the accommodation space 110i of FIG. 1) in which an article 10 may be accommodated or inserted, and an inner space (or a mounting space) in which the components of the brain stimulation device 100 may be arranged. For example, the processor 130, the battery 140, and the output unit 150 may be arranged in the inner space of the housing 110, but are not limited thereto.

The processor 130 may control the overall operation of the brain stimulation device 100. For example, the processor 130 may be implemented as an array of a plurality of logical gates, or may be implemented as a combination of a general-purpose microprocessor and a memory storing a program that may be executed by the general-purpose microprocessor. In addition, those skilled in the art to which the present embodiment belongs may understand that the processor 130 may be implemented in other forms of hardware.

According to an embodiment, the processor 130 may detect a type of the article 10 accommodated or inserted in the housing 110, and may control a current applied from the output unit 150 to a body B of a user. In the disclosure, the expression "a processor controls a current applied from an output unit to a body of a user" may indicate that the processor may control an intensity and/or frequency of the current applied from the output unit 150 to the body B of the user, and the corresponding expression may be used in the same sense below.

For example, when detecting that a first article is accommodated in the housing 110, the processor 130 may control the output unit 150 to apply a current corresponding to a first current profile to the body B of the user. As another example, when detecting that a second article different from the first article is accommodated in the housing 110, the processor 130 may control the output unit 150 to apply, to the body B of the user, a current corresponding to a second current profile different from the first current profile. In the disclosure, a current profile may refer to a change in current over time, and the corresponding expression may be used in the same sense hereinafter. The terms "first article" described in the specification mean article for providing the first current profile to the user, and the terms "second article" described in the specification mean article for providing the second current profile different from the first current profile to the user.

The processor 130 may provide the user with brain stimulation that enables the user to feel a smoking sensation by applying a current to the body B of the user via the output unit 150. Accordingly, via brain stimulation applied from the brain stimulation device 100, the user may feel substantially the same sensory and psychological stability as a smoking behavior, or may relieve stress or tension. A detailed description of the operation of the processor 130 controlling the output unit 150 will be given below.

The battery 140 may supply power used for the brain stimulation device 100 to operate. In an example, the battery 140 may supply power needed for the operation of the processor 130. In an example, the battery 140 may supply power needed for the output unit 150 to apply a current to the body B of the user. The battery 140 may be a rechargeable battery or a disposable battery. For example, the battery 140 may be a lithium polymer (LiPoly) battery, but the type of the battery 140 is not limited thereto.

The output unit 150 may generate stimulation to the brain of the user by applying a current to the body B of the user via power supplied from the battery 140. In other words, the output unit 150 may provide brain stimulation to the user by using power supplied from the battery 140.

For example, the output unit 150 may include at least one electrode (not shown) for generating a microcurrent via power supplied from the battery 140. At least one area of the output unit 150 may be in contact with the body B of the user, and the microcurrent generated from the at least one electrode may be applied to the body B of the user through the one area of the output unit 150 that is in contact with the body B of the user. The microcurrent generated by the output unit 150 may be applied to a brain circuit of the user, and thus, stimulation may occur in the brain of the user. Here, the output unit 150 may apply the microcurrent to the body B of the user so that brain stimulation corresponding to a smoking behavior may be applied to the user, and as a result, the user may feel a smoking sensation or psychological stability, or relieve stress or tension even without a separate smoking behavior.

According to an embodiment, the processor 130 may detect a type of the article 10 accommodated or inserted in the housing 110, and may control a current applied from the output unit 150 to the body B of the user on the basis of the detected type of the article 10. For example, the processor 130 may control an intensity or frequency of a current applied from the output unit 150 to the body B of the user.

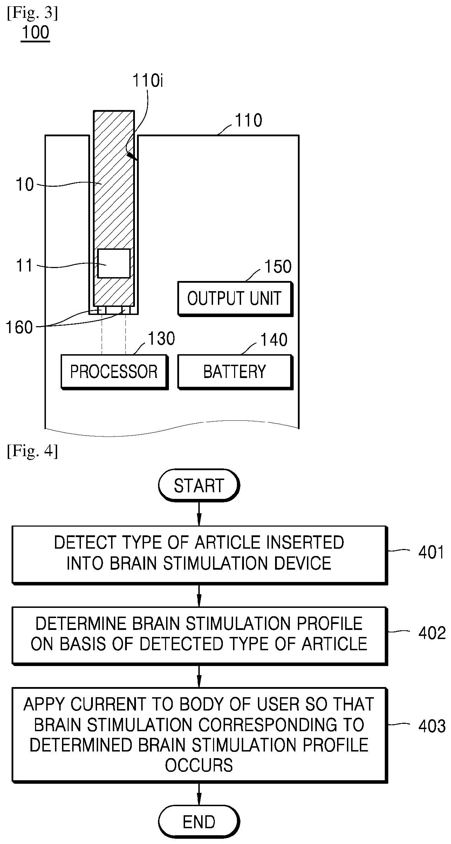

As a result, the processor 130 may provide various types of brain stimulation to the user according to the type of the article 10, and hereinafter, the process of detecting, by the processor 130, the type of the article 10 accommodated or inserted into the housing 110 will be described in detail with reference to FIG. 3.

FIG. 3 is a cross-sectional view illustrating an electrical connection relationship between a brain stimulation device as illustrated in FIG. 1 and an article accommodated in the brain stimulation device.

Referring to FIG. 3, a brain stimulation device 100 according to an embodiment may include a housing 110, a processor 130, a battery 140, an output unit 150, and at least one electrical connection unit 160. At least one of the components of the brain stimulation device 100 according to an embodiment may be the same as or similar to at least one of the components of the brain stimulation device 100 of FIG. 1 or 2, and the same description thereof will be omitted below.

The at least one electrical connection unit 160 may be arranged in an accommodation space 110i of the housing 110, and may contact an article 10 when the article 10 is accommodated or inserted in the accommodation space 110i. For example, the at least one electrical connection unit 160 may be electrically connected to a memory 11 embedded in the article 10 by contacting the article 10 when the article 10 is accommodated or inserted in the accommodation space 110i.

For example, the at least one electrical connection unit 160 may include a pogo-pin, but the type of the at least one electrical connection unit 160 is not limited thereto. In an example, the at least one electrical connection unit 160 may also include at least one electrode or a flexible printed circuit board (FPCB).

In the disclosure, the article 10 may be a component having the memory 11 embedded therein, and the memory 11 may store identification information of the article 10. In the disclosure, the identification information of the article 10 may refer to information indicating a type of the article 10. For example, the identification information of the article 10 may include information indicating whether the article 10 is a first article or a second article different from the first article, but is not limited thereto. In addition, FIG. 3 illustrates only the embodiment in which the article 10 has a stick shape, but the shape of the article 10 is not limited to the stick shape.

The processor 130 may be electrically connected to the at least one electrical connection unit 160, and thus may be electrically connected to the memory 11 of the article 10 via the at least one electrical connection unit 160 when the article 10 is accommodated or inserted in the accommodation space 110i. In other words, the processor 130 may be electrically connected to the memory 11 of the article 10 via the at least one connection unit 160.

The processor 130 may receive a signal including the identification information of the article 10 from the memory 11 of the article 10 via the at least one electrical connection unit 160, and may detect, on the basis of the received signal, a type of the article 10 accommodated or inserted in the accommodation space 110i.

According to an embodiment, the processor 130 may adjust, on the basis of the detected type of the article 10, an intensity of a current applied from the output unit 150 to a body of a user (e.g., the body B in FIG. 2). Here, according to the detected type of the article 10, the processor 130 may adjust the intensity of the current applied from the output unit 150 to the body of the user by controlling power supplied from the battery 140 to the output unit 150.

In an example, when detecting that the article 10 accommodated in the accommodation space 110i is a first article, the processor 130 may control the output unit 150 to apply brain stimulation corresponding to a first brain stimulation profile to the user. In an example, when detecting that the article 10 accommodated in the accommodation space 110i is a second article different from the first article, the processor 130 may control the output unit 150 to apply, to the user, brain stimulation corresponding to a second brain stimulation profile different from the first brain stimulation profile.

In the disclosure, a brain stimulation profile may refer to a change in brain stimulation applied to a user over time. For example, when stimulation corresponding to a first brain stimulation profile is applied to a user, the user may feel the smoking sensation as smoking a first article. Similarly, when stimulation corresponding to a second brain stimulation profile is applied to the user, the user may feel the same smoking sensation as smoking a second article.

The brain stimulation device 100 according to an embodiment may provide various smoking sensations or stress or tension relief effects to the user by providing different brain stimulation to the user according to the type of the article 10 accommodated in the accommodation space 110i. Accordingly, the user may feel various smoking sensations or reduce stress or tension via brain stimulation applied from the brain stimulation device 100, and thus may obtain the same effect as smoking even without a separate smoking behavior.

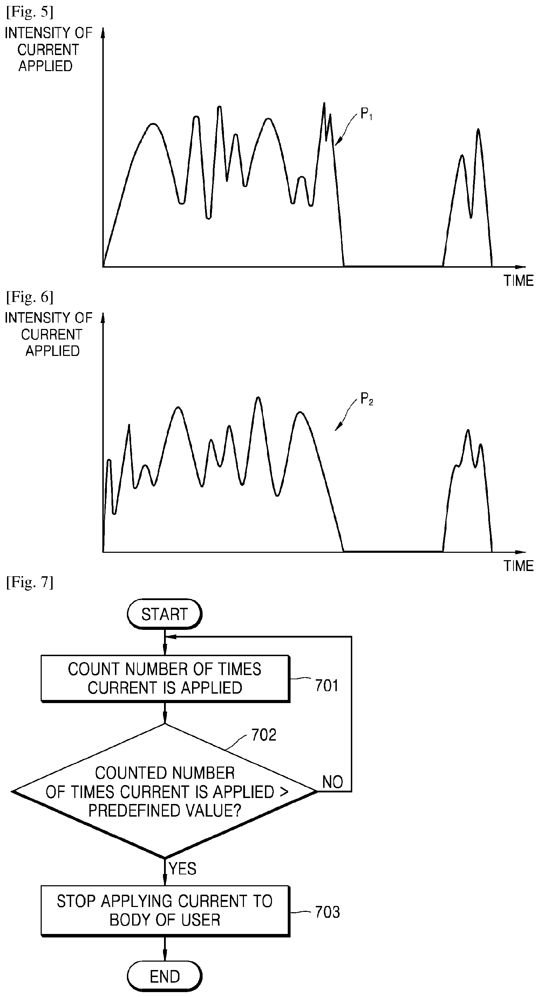

FIG. 4 is a flowchart illustrating operations of a brain stimulation device applying a current on the basis of a type of an article, according to an embodiment. In addition, FIG. 5 is a graph illustrating an example of a change in current applied to the body of a user when a first article is inserted into a brain stimulation device, and FIG. 6 is a graph illustrating an example of a change in current applied to the body of the user when a second article is inserted into the brain stimulation device.

Hereinafter, operations of a brain stimulation device applying a current, illustrated in FIG. 4, will be described with reference to the components of the brain stimulation device 100 illustrated in FIGS. 1 to 3 and current profiles P₁ and P₂ illustrated in FIGS. 5 and 6.

Referring to FIG. 4, in operation 401, the processor 130 of the brain stimulation device 100 according to an embodiment may detect a type of the article 10 accommodated or inserted in the brain stimulation device 100.

According to an embodiment, the processor 130 may detect the type of the article 10 accommodated in the accommodation space 110i via the at least one electrical connection unit 160 that is arranged in the accommodation space 110i and electrically connected to the memory 11 of the article 10 accommodated in the accommodation space 110i. For example, the processor 130 may receive a signal including identification information of the article 10 from the memory 11 of the article 10 via the at least one electrical connection unit 160, and may detect the type of the article 10 on the basis of the received signal.

In operation 402, the processor 130 of the brain stimulation device 100 according to an embodiment may determine, on the basis of the type of the article 10 detected in operation 401, a brain stimulation profile to be applied to the user.

In an example, when detecting that a first article is accommodated in the accommodation space 110i, the processor 130 may determine a first brain stimulation profile corresponding to the first article. In an example, when detecting that a second article different from the first article is accommodated in the accommodation space 110i, the processor 130 may determine a second brain stimulation profile corresponding to the second article.

Referring to FIGS. 4, 5, and 6, in operation 403, the processor 130 of the brain stimulation device 100 according to an embodiment may apply a current to the body B of the user via the output unit 150 so that brain stimulation corresponding to the brain stimulation profile determined in operation 402 is applied to the user.

Referring to FIG. 5, when detecting that the first article is accommodated in the accommodation space 110i, the processor 130 may apply a microcurrent corresponding to a first current profile P₁ to the body B of the user via the output unit 150 so that brain stimulation corresponding to the first brain stimulation profile is applied to the user. Here, the first current profile P₁ may refer to a change in current for providing the user with brain stimulation corresponding to the first brain stimulation profile.

In other words, when the first article is accommodated in the accommodation space 110i, the processor 130 may apply a current corresponding to the first current profile P1 to the body B of the user via the output unit 150, and as a result, brain stimulation corresponding to the first brain stimulation profile may occur in the brain of the user.

Referring to FIG. 6, when detecting that the second article is accommodated in the accommodation space 110i, the processor 130 may apply a microcurrent corresponding to a second current profile P₂ to the body B of the user via the output unit 150 so that brain stimulation corresponding to a second brain stimulation profile different from the first brain stimulation profile is applied to the user. Here, the second current profile P₂ may be distinguished from the first current profile P₁, and may refer to a change in current for providing the user with brain stimulation corresponding to the second brain stimulation profile.

In other words, when the second article is accommodated in the accommodation space 110i, the processor 130 may apply a current corresponding to the second current profile P₂ to the body B of the user via the output unit 150, and as a result, brain stimulation corresponding to the second brain stimulation profile different from the first brain stimulation profile may occur in the brain of the user.

The brain stimulation device 100 according to an embodiment may provide, via operations 401 to 403 described above, different brain stimulation to the user according to the type of the article 10 accommodated in the accommodation space 110i, and as a result, the user may feel various kinds of smoking sensations or psychological stability or relieve tension or stress with only brain stimulation without a separate smoking behavior.

FIG. 7 is a flowchart illustrating operations of a brain stimulation device controlling a current applied to the body of a user on the basis of the number of times a current is applied, according to an embodiment.

Hereinafter, operations of controlling a current applied to the body of a user, illustrated in FIG. 7, will be described with reference to the components of the brain stimulation device 100 illustrated in FIGS. 1 to 3.

Referring to FIG. 7, in operation 701, the processor 130 of the brain stimulation device 100 according to an embodiment may count the number of times the output unit 150 applies a current to the body B of the user. For example, the processor 130 may count the number of times the output unit 150 applies the current to the body B of the user on the basis of a time for which the battery 140 supplies power to the output unit 150, the number of times the battery 140 supplies power to the output unit 150, and/or an amount of power supplied from the battery 140 to the output unit 150.

In operation 702, the processor 130 of the brain stimulation device 100 according to an embodiment may determine whether or not the number of times the current is applied, counted in operation 701, is greater than a predefined value (or a predefined number of times). In the disclosure, the predefined value may refer to the maximum number of times a current may be applied to the body of a predefined user according to the article 10, and the predefined value described above may be a value stored in the memory 11 of the article 10 or a value stored in the processor 130. When the predefined value is stored in the memory 11 of the article 10, the processor 130 may receive data regarding the predefined value from the memory 11 via the at least one electrical connection unit 160.

In operation 703, when determining, in operation 702, that the counted number of times the current is applied is greater than the predefined value, the processor 130 of the brain stimulation device 100 according to an embodiment may stop applying the current to the body B of the user. For example, when the counted number of times the current is applied is greater than the predefined value, the processor 130 may stop the operation of the output unit 150 to stop applying the current to the body B of the user even when a user input to the at least one button unit 120 is received.

In contrast, when the counted number of times the current is applied is less than or equal to the predefined value in operation 702, the current may be additionally applied to the body B of the user, and thus, the processor 130 of the brain stimulation device 100 according to an embodiment may repeatedly perform operations 701 and 702 without stopping applying the current.

Via operations 701 to 703, the brain stimulation device 100 according to an embodiment may prevent the current from being applied to the user more than the predefined number of times, and accordingly, may prevent excessive brain stimulation from being applied to the user.

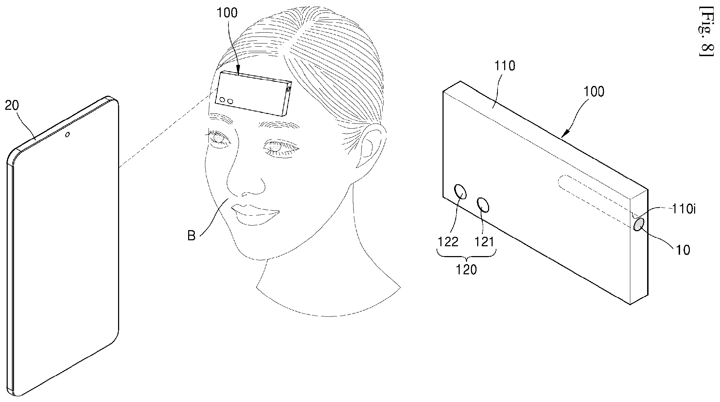

FIG. 8 is a perspective view illustrating a brain stimulation device and an external electronic device, according to an embodiment.

Referring to FIG. 8, a brain stimulation device 100 according to an embodiment may include a housing 110 including an accommodation space 110i having an article 10 accommodated therein, at least one button unit 120 for receiving an input of a user, and a communicator (not shown) for communication with an external device 20. The brain stimulation device 100 according to an embodiment may be a device to which the communicator is added to the brain stimulation device 100 illustrated in FIGS. 1 to 3, and the same description thereof will be omitted below.

The brain stimulation device 100 may be connected to the external device 20 by a wire and/or wirelessly via the communicator. Accordingly, the brain stimulation device 100 may transmit a signal to the external device 20 via the communicator, or may receive a signal from the external device 20 via the communicator.

The communicator may include at least one component for communication between the brain stimulation device 100 and the external device 20. For example, the communicator may include a short-range wireless communication unit and a wireless communication unit.

In an example, the short-range wireless communication unit may include a Bluetooth communication unit, a Bluetooth low energy (BLE) communication unit, a near field communication unit, a wireless local area network (WLAN (Wi-Fi)) communication unit, a Zigbee communication unit, an infrared data association (IrDA) communication unit, a Wi-Fi Direct (WFD) communication unit, a ultra wideband (UWB) communication unit, an Ant+ communication unit, and the like, but is not limited thereto. In an example, the wireless communication unit may include a cellular network communication unit, an Internet communication unit, a computer network (e.g., a local area network (LAN) or wide area network (WAN)) communication unit, and the like, but is not limited thereto.

FIG. 8 illustrates only an embodiment in which the brain stimulation device 100 is wirelessly connected to the external device 20 having the form of a mobile electronic device, but the embodiment is not limited thereto. According to embodiments, the brain stimulation device 100 may be connected to the external device 20 by a wire, or may be connected to an electronic device other than a mobile electronic device.

A processor (e.g., the processor 130 in FIGS. 2 and 3) of the brain stimulation device 100 according to an embodiment may control the overall operation of the brain stimulation device 100 on the basis of a signal received from the external device 20 by a wire or wirelessly. The signal received from the external device 20 may include data for controlling the operation of the brain stimulation device 100, and the processor may control the operation of the brain stimulation device 100 on the basis of the data included in the received signal.

In an example, on the basis of the signal received from the external device 20, the processor of the brain stimulation device 100 may switch power of the brain stimulation device 100 from an on state to an off state or from the off state to the on state.

In an example, the processor of the brain stimulation device 100 may adjust, on the basis of the signal received from the external device 20, an intensity of a current applied to a user from an output unit (e.g., the output unit 150 in FIG. 2) of the brain stimulation device 100.

In other words, the brain stimulation device 100 according to an embodiment may control the overall operation of the brain stimulation device 100 on the basis of an input of the user to the external device 20 as well as an input of the user to the at least one button unit 120. Accordingly, the user may control the brain stimulation device 100 in various methods, and thus, the convenience of use of the brain stimulation device 100 may be improved.

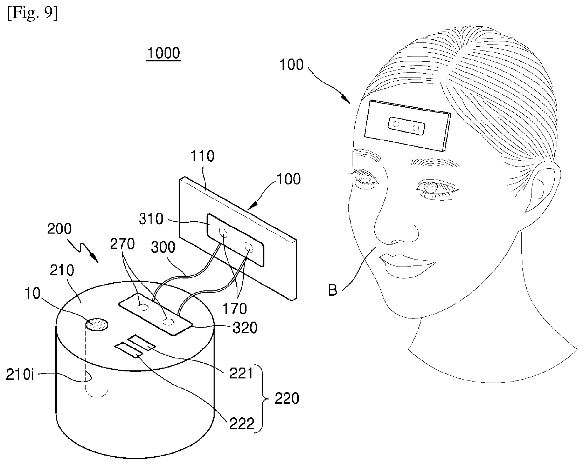

FIG. 9 is a perspective view illustrating a brain stimulation system according to an embodiment.

Referring to FIG. 9, a brain stimulation system 1000 according to an embodiment may include a brain stimulation device 100 attached to a body B of a user and a control device 200 (or a control module) for controlling the brain stimulation device 100.

The brain stimulation device 100 may be attached to at least one area of the body B of the user to apply a microcurrent, and thus may provide the user with brain stimulation corresponding to a smoking sensation.

According to an embodiment, the brain stimulation device 100 may include a first housing 110 (e.g., the housing 110 in FIG. 1) and an output unit (not shown) (e.g., the output unit 150 in FIG. 2).

The first housing 110 may form the overall appearance of the brain stimulation device 100, and may have formed therein an inner space (or a mounting space) in which components of the brain stimulation device 100 may be arranged. For example, a processor (e.g., the processor 130 in FIG. 2), a battery (e.g., the battery 140 in FIG. 2), and the output unit may be arranged in the inner space of the first housing 110, but are not limited thereto.

FIG. 9 illustrates only an embodiment in which the first housing 110 is formed to have a rectangular patch shape as a whole, but the shape of the first housing 110 is not limited thereto. In an example, the first housing 110 may be formed to have an elliptical patch shape, or may be formed to have a polygonal patch shape.

The output unit may provide brain stimulation to the user by applying a current to the body B of the user on the basis of power supplied. For example, the output unit may apply the current to the body B of the user through at least one area of the first housing 110 that is in contact with the body B of the user.

The control device 200 may be operatively connected to the brain stimulation device 100, and may control the overall operation of the brain stimulation device 100. In other words, the user may adjust, via the control device 200, a power state of the brain stimulation device 100 and/or an intensity of a current applied from the brain stimulation device 100 to the body B, a frequency of the current, or the like.

According to an embodiment, the control device 200 may include a second housing 210 including an accommodation space 210i having an article 10 accommodated or inserted therein, and at least one button unit 220 for receiving an input of the user.

The second housing 210 may form the overall appearance of the control device 200, and the second housing 210 may have one area having arranged therein the accommodation space 210i in which at least a portion of the article 10 may be accommodated or inserted. The control device 200 may detect a type of the article 10 accommodated or inserted in the accommodation space 210i of the second housing 210, and may transmit data corresponding to the detected type of the article 10 to the brain stimulation device 100. The brain stimulation device 100 may control a current applied to the body B of the user on the basis of the data regarding the type of the article 10, received from the control device 200, and a detailed description thereof will be given below.

FIG. 9 illustrates only an embodiment in which the second housing 210 is formed to have a cylindrical shape as a whole, but the shape of the second housing 210 is not limited thereto. According to embodiments, the second housing 210 may be formed to have a polygonal pillar shape (e.g., a rectangular pillar shape or a pentagonal pillar shape).

The at least one button unit 220 may be arranged on an outer circumferential surface of the second housing 210 to receive a user input. A processor (not shown) of the control device 200 may transmit, to the brain stimulation device 100, a signal for controlling the operation of the brain stimulation device 100, on the basis of an input of the user to the at least one button unit 220.

According to an embodiment, the at least one button unit 220 may include a first button unit 221 for controlling a power state of the brain stimulation device 100 and a second button unit 222 for controlling an intensity or frequency of brain stimulation and/or current applied from the brain stimulation device 100 to the user. However, the number of at least one button unit 220 is not limited to the embodiment described above, and the number of button units may increase or decrease according to embodiments.

In an example, when a user input to the first button unit 221 is received, the processor of the control device 200 may transmit, to the brain stimulation device 100, a signal including data for controlling the power state of the brain stimulation device 100. On the basis of the signal received from the control device 200, the brain stimulation device 100 may switch the power state from an on state to an off state, or conversely, from the off state to the on state.

In an example, when an input of the user to the second button unit 222 is received, the processor of control device 200 may transmit a signal including data for adjusting an intensity or frequency of a current applied from the brain stimulation device 100 to the user. For example, on the basis of the signal received from the control device 200, the brain stimulation device 100 may adjust the intensity or frequency of the current applied to the user so that strong brain stimulation may be applied to the user or brain stimulation may be applied to the user more quickly. In addition, on the basis of the signal received from the control device 200, the brain stimulation device 100 may adjust the intensity or frequency of the current applied to the user so that weaker brain stimulation may be applied to the user or brain stimulation may be applied to the user more slowly.

According to an embodiment, the brain stimulation system 1000 may further include a connector 300 for electrically or operatively connecting the brain stimulation device 100 to the control device 200.

For example, one end 310 of the connector 300 may be connected to a first electrode 170 of the brain stimulation device 100, and the other end 320 of the connector 300 may be connected to a second electrode 270 of the control device 200. Here, the first electrode 170 may be a component arranged in one area of the first housing 110 of the brain stimulation device 100 and electrically connecting the connector 300 to the processor of the brain stimulation device 100. In addition, the second electrode 270 may be a component arranged in one area of the second housing 210 of the control device 200 and electrically connecting the connector 300 to the processor of the control device 200.

The connector 300 may be, for example, a wire or a FPCB, but is not limited thereto. In an example, the connector 300 may also be an optical axis cable.

The brain stimulation device 100 and the control device 200 may be electrically or operatively connected to each other via the connector 300, and a signal may be transmitted between the brain stimulation device 100 and the control device 200 via the connector 300.

According to an embodiment, the brain stimulation device 100 and the control device 200 may be wirelessly connected to each other without a separate component (e.g., the connector 300) for connecting the brain stimulation device 100 and the control device 200 to each other.

For example, the brain stimulation device 100 may include a first communicator for communicating with the control device 200, and the control device 200 may include a second communicator for communicating with the brain stimulation device 100. The brain stimulation device 100 and the control device 200 may be electrically or operatively connected to each other via the first communicator and the second communicator, and as a result, a signal may be transmitted between the brain stimulation device 100 and the control device 200.

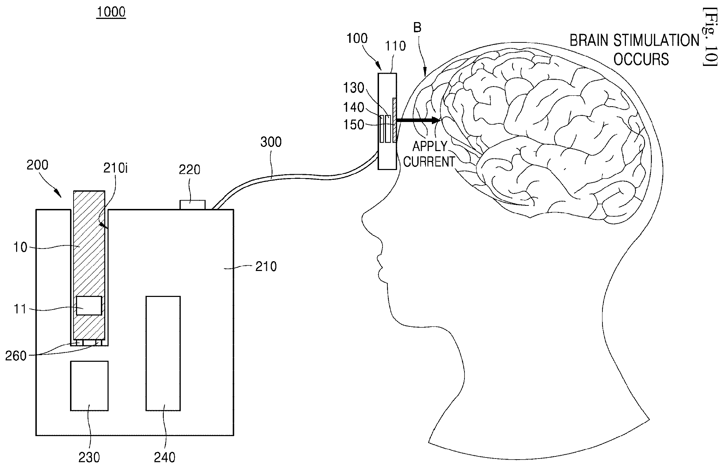

Hereinafter, the components of the brain stimulation system 1000 according to an embodiment will be described in detail with reference to FIG. 10.

FIG. 10 is a diagram illustrating a process of applying, by a brain stimulation system as illustrated in FIG. 9, a current to the body of a user.

Referring to FIG. 10, a brain stimulation system 1000 according to an embodiment may include a brain stimulation device 100 attached to a body B of a user to apply a current, a control device 200 for controlling the brain stimulation device 100, and a connector 300 for connecting the brain stimulation device 100 to the control device 200. At least one of the components of the brain stimulation system 1000 according to an embodiment may be the same as or similar to at least one of the components of the brain stimulation system 1000 of FIG. 9, and the same description thereof will be omitted below.

In addition, the components of the brain stimulation system 1000 are not limited thereto, and according to embodiments, other components may be added, or at least one (e.g., the connector 300) of the components described above may be omitted.

The brain stimulation device 100 may include a first housing 110, a first processor 130, a first battery 140, and an output unit 150.

The first housing 110 may have at least one area attached to the body B of the user, and may have formed therein an inner space in which the components of the brain stimulation device 100 may be arranged. For example, the first processor 130, the first battery 140, and the output unit 150 may be arranged in the inner space of the first housing 110, but are not limited thereto.

The first processor 130 may be electrically connected to the first battery 140 and the output unit 150 to control the overall operation of the brain stimulation device 100. For example, the first processor 130 may be implemented as an array of a plurality of logical gates, or may be implemented as a combination of a general-purpose microprocessor and a memory storing a program that may be executed by the general-purpose microprocessor. In addition, those skilled in the art to which the embodiment belongs may understand that the first processor 130 may be implemented in other forms of hardware.

In an example, the first processor 130 may control a power state of the brain stimulation device 100 on the basis of a signal received from the control device 200. For example, on the basis of the signal received from the control device 200, the first processor 130 may switch the power state of the brain stimulation device 100 from an on state to an off state, or conversely, from the off state to the on state.

In an example, on the basis of the signal received from the control device 200, the first processor 130 may adjust an intensity or frequency of a current applied to the body B of the user via the output unit 150 by controlling power supplied from the first battery 140 to the output unit 150. For example, on the basis of the signal received from the control device 200, the first processor 130 may adjust the intensity or frequency of the current applied to the user so that strong brain stimulation may be applied to the user or brain stimulation may be applied to the user more quickly. In addition, on the basis of the signal received from the control device 200, the first processor 130 may adjust the intensity or frequency of the current applied to the user so that weaker brain stimulation may be applied to the user or brain stimulation may be applied to the user more slowly.

The first battery 140 may supply power used for the brain stimulation device 100 to operate. In an example, the first battery 140 may supply power needed for the operation of the first processor 130. In an example, the first battery 140 may supply power needed for the output unit 150 to apply a current to the body B of the user. The first battery 140 may be a rechargeable battery or a disposable battery. For example, the first battery 140 may be a lithium polymer (LiPoly) battery, but the type of the first battery 140 is not limited thereto.

The output unit 150 may generate stimulation to the brain of the user by applying a current to the body B of the user via power supplied from the first battery 140. In other words, the output unit 150 may provide brain stimulation to the user by using power supplied from the first battery 140.

For example, the output unit 150 may include at least one electrode (not shown) for generating a microcurrent via power supplied from the first battery 140. At least one area of the output unit 150 may be in contact with the body B of the user, and the microcurrent generated from the at least one electrode may be applied to the body B of the user through the one area of the output unit 150 that is in contact with the body B of the user. Here, the microcurrent generated by the output unit 150 may be applied to a brain circuit of the user, and thus, stimulation may occur in the brain of the user.

The control device 200 (or a control module) may include a second housing 210, at least one button unit 220, a second processor 230, a second battery 240, and at least one electrical connection unit 260.

The second housing 210 may form the overall appearance of the control device 200, and may include an accommodation space 210i in which an article 10 may be accommodated or inserted, and an inner space (or a mounting space) in which the components of the control device 200 may be arranged. For example, the second processor 230 and the second battery 240 may be arranged in the inner space of the second housing 210, but are not limited thereto.

According to an embodiment, the second housing 210 may be formed to have a size and/or shape that is easily gripped by the user. For example, the second housing 210 may be formed to have a cylindrical shape that is easily gripped by the user, but the shape of the second housing 210 is not limited thereto.

The second processor 230 may control the overall operation of the control device 200. For example, the second processor 230 may detect a type of an article 10 accommodated or inserted in the accommodation space 210i, and may transmit, to the brain stimulation device 100, a signal including data corresponding to the detected type of the article 10.

According to an embodiment, the second processor 230 may transmit, to the first processor 130 of the brain stimulation device 100 via a connector 300, a signal including data corresponding to the type of the article 10 accommodated or inserted in the accommodation space 210i, but is not limited thereto. In an embodiment, the second processor 230 may also transmit the signal including the data corresponding to the type of article 10 accommodated or inserted in the accommodation space 210i to the first processor 130 of the brain stimulation device 100 via wireless communication without the connector 300.

In addition, the second processor 230 may transmit, to the first processor 130 on the basis of an input of the user to the at least one button unit 220, a signal including data for controlling a power state of the brain stimulation device 100 or an intensity or frequency of a current applied from the output unit 150 of the brain stimulation device 100 to the body B of the user.

On the basis of the signal received from the second processor 230, the first processor 130 may switch the power state of the brain stimulation device 100 to an on state or an off state, or may adjust the intensity or frequency of the current applied from the output unit 150 to the body B of the user.

The second battery 240 may supply power used for the control device 200 to operate. For example, the second battery 240 may supply power needed for the operation of the second processor 230. The second battery 240 may be a rechargeable battery or a disposable battery. For example, the second battery 240 may be a lithium polymer (LiPoly) battery, but the type of the second battery 240 is not limited thereto.

The at least one electrical connection unit 260 may be arranged in the accommodation space 210i of the second housing 210, and may contact the article 10 accommodated or inserted in the accommodation space 210i. For example, the at least one electrical connection unit 260 may be electrically connected to a memory 11 embedded in the article 10 by contacting the article 10 when the article 10 is accommodated or inserted in the accommodation space 210i.

The at least one electrical connection unit 260 may include, for example, a pogo-pin, but the type of the at least one electrical connection unit 260 is not limited thereto. In an example, the at least one electrical connection unit 260 may also include at least one electrode or a FPCB.

The second processor 230 may be electrically connected to the at least one electrical connection unit 260, and thus may be electrically connected to a memory 11 of the article 10 via the at least one electrical connection unit 260 when the article 10 is accommodated or inserted in the accommodation space 210i. In other words, the second processor 230 may be electrically connected to the memory 11 of the article 10 via the at least one electrical connection unit 260.

The second processor 230 may receive a signal including identification information of the article 10 from the memory 11 of the article 10 via the at least one electrical connection unit 260, and may detect, on the basis of the received signal, a type of the article 10 accommodated or inserted in the accommodation space 210i.

According to an embodiment, the second processor 230 may determine a brain stimulation profile corresponding to the detected type of the article 10, and transmit, to the first processor 130, a signal including data regarding the determined brain stimulation profile. On the basis of the signal received from the second processor 230, the first processor 130 may control a current applied from the output unit 150 to the body B of the user. In the disclosure, the expression "the first processor 130 controls the current applied from the output unit 150 to the body B of the user" indicates that the first processor 130 may control an intensity and/or frequency of the current applied from the output unit 150 to the body B of the user, and the corresponding expression may be used in the same sense below.

In an example, when detecting that a first article is accommodated in the accommodation space 210i, the second processor 230 may determine that stimulation corresponding to a first brain stimulation profile matching the first article needs to be applied to the user, and may transmit a signal including data regarding the first brain stimulation profile to the first processor 130. Here, on the basis of the signal received from the second processor 230, the first processor 130 of the brain stimulation device 100 may control a current applied from the output unit 150 to the body B of the user so that brain stimulation corresponding to the first brain stimulation profile may be applied to the user.

In an example, when detecting that a second article different from the first article is accommodated in the accommodation space 210i, the second processor 230 may determine that stimulation corresponding to a second brain stimulation profile matching the second article needs to be applied to the user, and may transmit a signal including data regarding the second brain stimulation profile to the first processor 130. Here, on the basis of the signal received from the second processor 230, the first processor 130 of the brain stimulation device 100 may control the current applied from the output unit 150 to the body B of the user so that brain stimulation corresponding to the second brain stimulation profile may be applied to the user.

In the disclosure, a brain stimulation profile may refer to a change in brain stimulation applied to a user over time. For example, when stimulation corresponding to a first brain stimulation profile is applied to a user, the user may feel the smoking sensation as smoking a first article. Similarly, when stimulation corresponding to a second brain stimulation profile is applied to the user, the user may feel the same smoking sensation as smoking a second article.

According to an embodiment, the first processor 130 may count the number of times a current is applied to the body B of the user via the output unit 150, and may control the operation of the output unit 150 on the basis of the counted number of times the current is applied. For example, when the counted number of times the current is applied is greater than a predefined value (or the predefined number of times), the first processor 130 may stop the operation of the output unit 150 so that the current is no longer applied to the body B of the user.

In the disclosure, the predefined value may refer to the maximum number of times a current may be applied to a predefined body of a user according to the article 10, and the predefined value described above may be a value stored in the memory 11 of the article 10, or may be a value stored in the first processor 130 or the second processor 230.

According to an embodiment, on the basis of the number of times a signal is received from the second processor 230, the first processor 130 may count the number of times a current is applied to the body B of the user via the output unit 150, and may control the operation of the output unit 150 on the counted number of times the current is applied.

In other words, the brain stimulation system 1000 according to an embodiment may provide various types of brain stimulation to the user by controlling the current applied from the brain stimulation device 100 to the user according to the type of the article 10 accommodated in the accommodation space 210i of the control device 200. Accordingly, the user may feel various smoking sensations and/or tension or stress relief effects only with brain stimulation without a separate smoking behavior.

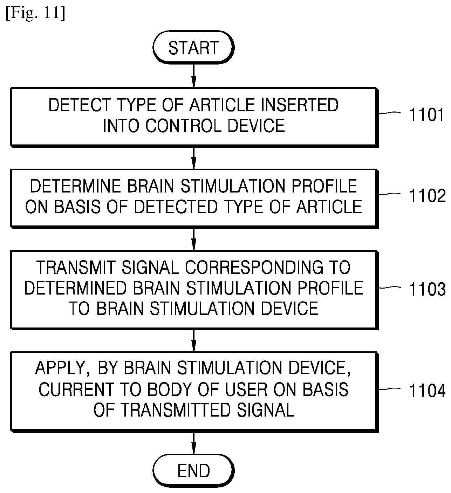

FIG. 11 is a flowchart illustrating operations of a brain stimulation system applying a current on the basis of a type of an article, according to an embodiment. Hereinafter, operations of a brain stimulation system applying a current, illustrated in FIG. 11, will be described with reference to the components of the brain stimulation system 1000 illustrated in FIGS. 9 and 10.

Referring to FIG. 11, in operation 1101, the control device 200 of the brain stimulation system 1000 according to an embodiment may detect a type of the article 10 accommodated or inserted in the accommodation space 210i.

According to an embodiment, the second processor 230 of the control device 200 may detect the type of the article 10 accommodated in the accommodation space 210i via the at least one electrical connection unit that is arranged in the accommodation space 210i and electrically connected to the memory 11 of the article 10 accommodated in the accommodation space 210i. For example, the second processor 230 may receive a signal including identification information of the article 10 from the memory 11 of the article 10 via the at least one electrical connection unit 260, and may detect the type of the article 10 on the basis of the received signal, but is not limited thereto.

In operation 1102, the control device 200 of the brain stimulation system 1000 according to an embodiment may determine a brain stimulation profile to be applied to the user, on the basis of the type of the article 10 detected in operation 1101.

In an example, when detecting that a first article is accommodated in the accommodation space 210i, the second processor 230 of the control device 200 may determine that brain stimulation corresponding to a first brain stimulation profile needs to be applied to the user. In an example, when detecting that a second article different from the first article is accommodated in the accommodation space 210i, the second processor 230 of the control device 200 may determine that brain stimulation corresponding to a second brain stimulation profile different from the first brain stimulation profile needs to be applied to the user.

In operation 1103, the control device 200 of the brain stimulation system 1000 according to an embodiment may transmit, to the brain stimulation device 100, a signal corresponding to the brain stimulation profile determined in operation 1102.

According to an embodiment, the control device 200 may be electrically or operatively connected to the brain stimulation device 100 via the connector 300, and may transmit, to the brain stimulation device 100, a signal including data regarding the brain stimulation profile determined in operation 1102.

In an example, when determining, in operation 1102, that brain stimulation corresponding to the first brain stimulation profile needs to be applied to the user, the control device 200 may transmit, to the brain stimulation device 100, a first signal including data regarding the first brain stimulation profile.

In an example, when determining, in operation 1102, that brain stimulation corresponding to the second brain stimulation profile needs to be applied to the user, the control device 200 may transmit, to the brain stimulation device 100, a second signal including data regarding the second brain stimulation profile.

According to an embodiment, the control device 200 may be electrically or operatively connected to the brain stimulation device 100 via wireless communication, and may also transmit, to the brain stimulation device 100, a wireless signal including data regarding the brain stimulation profile determined in operation 1102.

In operation 1104, on the basis of the signal received from the control device 200 via operation 1103, the brain stimulation device 100 of the brain stimulation system 1000 according to an embodiment may apply brain stimulation corresponding to a smoking sensation to the user by applying a current to the body B of the user via the output unit 150.

In an example, when receiving the first signal including the data regarding the first brain stimulation profile from the control device 200, the first processor 130 of the brain stimulation device 100 may apply a microcurrent corresponding to a first current profile (e.g., the first current profile P₁ in FIG. 5) to the body B of the user via the output unit 150 so that brain stimulation corresponding to the first brain stimulation profile may be applied to the user.

In an example, when receiving the second signal including the data regarding the second brain stimulation profile from the control device 200, the first processor 130 of the brain stimulation device 100 may apply a microcurrent corresponding to a second current profile (e.g., the second current profile P₂ in FIG. 6) to the body B of the user via the output unit 150 so that brain stimulation corresponding to the second brain stimulation profile is applied to the user.

The brain stimulation system 1000 according to an embodiment may provide different types of brain stimulation to the user according to the type of the article 10 accommodated in the accommodation space 210i via operations 1101 to 1104 described above, and as a result, the user may feel various smoking sensations, psychological stability, and/or stress or tension relief effects with only brain stimulation without a separate smoking behavior.

Operations of a brain stimulation device or a brain stimulation system according to various embodiments may be implemented in the form of a recording medium including computer-executable instructions, such as a program module executed by a computer. The computer-readable recording medium may be any available medium that may be accessed by a computer and includes both volatile and nonvolatile media, and removable and non-removable media. In addition, the computer-readable recording medium may include both a computer storage medium and a communication medium. The computer storage medium includes all of volatile and nonvolatile media, and removable and non-removable media implemented by any method or technology for storage of information such as computer-readable instructions, data structures, program modules, or other data. The communication medium typically includes computer-readable instructions, data structures, other data in modulated data signals such as program modules, or other transmission mechanisms, and includes any information transfer media.

## Claims

1. A brain stimulation device (100) comprising:
a housing (110) having at least one area configured to be attached to a body (B) of a user, and including an accommodation space (110i) for accommodating an article comprising a memory (11);
a battery (140) arranged inside the housing (110);
an output unit (150) configured to apply a current to the body (B) of the user based on power supplied from the battery (140); and
a processor (130) electrically connected to the battery (140) and the output unit (150), and configured to: detect a type of the article (10) accommodated in the accommodation space (110i); and based on the detected type of the article (10), apply a current to the body (B) of the user via the output unit (150) so that brain stimulation is applied to the user,
wherein the brain stimulation device (100) further comprises at least one electrical connection unit (260) arranged in the accommodation space (110), wherein the at least one electrical connection unit (260) is electrically connectable to the memory (11) of the article (10) when the article (10) is accommodated in the accommodation space (110i), and
wherein the processor (130) is configured to: receive identification information from the memory (11) of the article (10) via the at least one electrical connection unit (260) when the article (10) is accommodated in the accommodation space (110i); and based on the received identification information, detect the type of the article (10) accommodated in the accommodation space (110i).

2. The brain stimulation device of claim 1, further comprising at least one button unit (220) configured to receive an input of the user, wherein the processor (130) is configured to control power of the brain stimulation device (100) or an intensity of a current applied to the body (B) of the user, based on a user input to the at least one button unit (220).

3. The brain stimulation device of claim 1, wherein the processor (130) is configured to: when detecting that a first article is accommodated in the accommodation space (110i), apply a current corresponding to a first current profile to the body (B) of the user via the output unit (150) so that brain stimulation corresponding to a first brain stimulation profile is applied to the user; and when detecting that a second article different from the first article is accommodated in the accommodation space (110i), apply a current corresponding to a second current profile to the body of the user via the output unit (150) so that brain stimulation corresponding to a second brain stimulation profile is applied the user.

4. The brain stimulation device of claim 1, wherein the processor (130) is configured to: count a number of times a current is applied to the body of the user via the output unit (150); and stop an operation of the output unit (150) when the counted number of times is greater than a predefined value.

5. The brain stimulation device of claim 1, further comprising a communicator configured to transmit a signal to or receive a signal from an external device (20), wherein the processor (130) is configured to control an operation of the brain stimulation device (100), based on a signal received from the external device (20) via the communicator.

6. A brain stimulation system (1000) comprising:
a brain stimulation device (100) having at least one area attachable to a body (B) of a user; and
a control device (200) operatively connected to the brain stimulation device (100),
wherein the brain stimulation device (100) includes: a first housing (110) having the at least one area attachable to the body (B) of the user; a battery (140) arranged inside the first housing (110); an output unit (150) configured to apply a current to the body (B) of the user, based on power supplied from the battery (140); and a first processor (130) electrically connected to the battery (140) and the output unit (150), and
the control device (200) includes: a second housing (210) including an accommodation space (210i) for accommodating an article (10) comprising a memory (11); and a second processor (230) configured to detect a type of the article (10) accommodated in the accommodation space (210i),
wherein the first processor (130) is configured to: receive, from the second processor (230), a signal including data corresponding to the detected type of the article (10); and based on the received signal, apply a current to the body (B) of the user via the output unit (150) so that brain stimulation is applied to the user,
wherein the control device (200) further includes at least one electrical connection unit (260) arranged in the accommodation space (210i), wherein the at least one electrical connection unit (260) is electrically connectable to the memory (11) of the article (10) when the article (10) is accommodated in the accommodation space (210i), and
wherein the second processor (230) is configured to: receive identification information from the memory (11) of the article (10) via the at least one electrical connection unit (260) when the article (10) is accommodated in the accommodation space (210i); and based on the received identification information, detect the type of the article (10) accommodated in the accommodation space (210i).

7. The brain stimulation system of claim 6, further comprising a connector (300) having one end connected to the brain stimulation device (100) and another end connected to the control device (200), wherein the first processor (130) is configured to receive, from the second processor (230) via the connector (300), a signal including data corresponding to the detected type of the article (10).

8. The brain stimulation system of claim 6, wherein the brain stimulation device (100) further includes a first communicator, the control device (200) further includes a second communicator for communication with the first communicator, and the first processor (130) is configured to receive, from the second processor (230) via the first communicator and the second communicator, a signal including data corresponding to the detected type of the article (10).

9. The brain stimulation system of claim 6, wherein the control device (200) further includes at least one button unit (220), wherein the second processor (230) is configured to transmit, to the first processor (130), a signal for controlling power of the brain stimulation device (100) or an intensity of a current applied from the output unit (150) to the body (B) of the user, based on a user input to the at least one button unit (220).

10. The brain stimulation system of claim 6, wherein the second processor (230) is configured to transmit, to the first processor (130), a first signal including data indicating that a first article (10) is accommodated, when detecting that the first article (10) is accommodated in the accommodation space (210i), and the first processor (130) is configured to apply a current corresponding to a first current profile to the body (B) of the user via the output unit (150) so that brain stimulation corresponding to a first brain stimulation profile is applied to the user, based on the first signal received from the second processor (230).

11. The brain stimulation system of claim 10, wherein the second processor (230) is configured to transmit, to the first processor (130), a second signal including data indicating that a second article is accommodated, when detecting that the second article different from the first article (10) is accommodated in the accommodation space (210i), and the first processor (130) is configured to apply a current corresponding to a second current profile to the body (B) of the user via the output unit (150) so that brain stimulation corresponding to a second brain stimulation profile is applied to the user, based on the second signal received from the second processor (230).

## Patentansprüche

1. Gehirnstimulationsvorrichtung (100), die Folgendes umfasst:
ein Gehäuse (110), das mindestens einen Bereich aufweist, der konfiguriert ist, am Körper (B) eines Benutzers befestigt zu sein, und das einen Aufnahmeraum (110i) zum Aufnehmen eines Gegenstands, der einen Speicher (11) umfasst, enthält;
eine Batterie (140), die im Inneren des Gehäuses (110) angeordnet ist;
eine Ausgangseinheit (150), die konfiguriert ist, auf der Grundlage von Energie, die von der Batterie (140) zugeführt wird, einen Strom in den Körper (B) des Benutzers einzugeben; und
einen Prozessor (130), der mit der Batterie (140) und der Ausgangseinheit (150) elektrisch verbunden ist und konfiguriert ist zum: Detektieren eines Typs des Gegenstands (10), der im Aufnahmeraum (110i) aufgenommen ist; und auf der Grundlage des detektierten Typs des Gegenstands (10) Eingeben eines Stroms in den Körper (B) des Benutzers über die Ausgangseinheit (150), derart, dass eine Gehirnstimulation auf den Benutzer angewendet wird,
wobei die Gehirnstimulationsvorrichtung (100) ferner mindestens eine elektrische Verbindungseinheit (260) umfasst, die im Aufnahmeraum (110) angeordnet ist, wobei die mindestens eine elektrische Verbindungseinheit (260) mit dem Speicher (11) des Gegenstands (10) elektrisch verbindbar ist, wenn der Gegenstand (10) im Aufnahmeraum (110i) aufgenommen ist, und
wobei der Prozessor (130) konfiguriert ist zum: Empfangen von Identifikationsinformationen vom Speicher (11) des Gegenstands (10) über die mindestens eine elektrische Verbindungseinheit (260), wenn der Gegenstand (10) im Aufnahmeraum (110i) aufgenommen ist; und auf der Grundlage der empfangenen Identifikationsinformationen, Detektieren des Typs des Gegenstands (10), der im Aufnahmeraum (110i) aufgenommen ist.

2. Gehirnstimulationsvorrichtung nach Anspruch 1, die ferner mindestens eine Knopfeinheit (220) umfasst, die konfiguriert ist, eine Eingabe des Benutzers zu empfangen, wobei der Prozessor (130) konfiguriert ist, die Leistung der Gehirnstimulationsvorrichtung (100) oder eine Intensität eines Stroms, der in den Körper (B) des Benutzers eingegeben wird, auf der Grundlage einer Benutzereingabe in die mindestens eine Knopfeinheit (220) zu steuern.

3. Gehirnstimulationsvorrichtung nach Anspruch 1, wobei der Prozessor (130) konfiguriert ist zum: Eingeben eines Stroms, der einem ersten Stromprofil entspricht, über die Ausgangseinheit (150) in den Körper (B) des Benutzers, wenn detektiert wird, dass ein erster Gegenstand im Aufnahmeraum (110i) aufgenommen ist, derart, dass eine Gehirnstimulation, die einem ersten Gehirnstimulationsprofil entspricht, auf den Benutzer angewendet wird; und Eingeben eines Stroms, der einem zweiten Stromprofil entspricht, über die Ausgangseinheit (150) in den Körper des Benutzers, wenn detektiert wird, dass ein zweiter Gegenstand, der vom ersten Gegenstand verschieden ist, im Aufnahmeraum (110i) aufgenommen ist, derart, dass eine Gehirnstimulation, die einem zweiten Gehirnstimulationsprofil entspricht, auf den Benutzer angewendet wird.

4. Gehirnstimulationsvorrichtung nach Anspruch 1, wobei der Prozessor (130) konfiguriert ist zum: Zählen einer Anzahl von Wiederholungen, für die über die Ausgangseinheit (150) ein Strom in den Körper des Benutzers eingegeben wird; und Anhalten eines Betriebs der Ausgangseinheit (150), wenn die gezählte Anzahl der Wiederholungen größer als ein vorgegebener Wert ist.

5. Gehirnstimulationsvorrichtung nach Anspruch 1, die ferner eine Kommunikationseinrichtung umfasst, die konfiguriert ist, ein Signal zu einer externen Vorrichtung (20) zu senden oder ein Signal von dieser zu empfangen, wobei der Prozessor (130) konfiguriert ist, einen Betrieb der Gehirnstimulationsvorrichtung (100) auf der Grundlage eines Signals, das über die Kommunikationseinrichtung von der externen Vorrichtung (20) empfangen wird, zu steuern.

6. Gehirnstimulationssystem (1000), das Folgendes umfasst:
eine Gehirnstimulationsvorrichtung (100), die mindestens einen Bereich aufweist, der am Körper (B) eines Benutzers befestigt werden kann; und
eine Steuervorrichtung (200), die mit der Gehirnstimulationsvorrichtung (100) betriebstechnisch verbunden ist,
wobei die Gehirnstimulationsvorrichtung (100) Folgendes enthält: ein erstes Gehäuse (110), das den mindestens einen Bereich aufweist, der am Körper (B) des Benutzers befestigt werden kann; eine Batterie (140), die im Inneren des ersten Gehäuses (110) angeordnet ist; eine Ausgangseinheit (150), die konfiguriert ist, auf der Grundlage von Energie, die von der Batterie (140) zugeführt wird, einen Strom in den Körper (B) des Benutzers einzugeben; und einen ersten Prozessor (130), der mit der Batterie (140) und der Ausgangseinheit (150) elektrisch verbunden ist, und
die Steuervorrichtung (200) Folgendes enthält: ein zweites Gehäuse (210), das einen Aufnahmeraum (210i) zum Aufnehmen eines Gegenstands (10), der einen Speicher (11) umfasst, enthält; und einen zweiten Prozessor (230), der konfiguriert ist, einen Typ des Gegenstands (10), der im Aufnahmeraum (230i) aufgenommen ist, zu detektieren,
wobei der erste Prozessor (130) konfiguriert ist zum: Empfangen eines Signals, das Daten enthält, die dem detektierten Typ des Gegenstands (10) entsprechen, vom zweiten Prozessor (230); und auf der Grundlage des empfangenen Signals Eingeben eines Stroms in den Körper (B) des Benutzers über die Ausgangseinheit (150), derart, dass eine Gehirnstimulation auf den Benutzer angewendet wird,
wobei die Steuervorrichtung (200) ferner mindestens eine elektrische Verbindungseinheit (260) enthält, die im Aufnahmeraum (210i) angeordnet ist, wobei die mindestens eine elektrische Verbindungseinheit (260) mit dem Speicher (11) des Gegenstands (10) elektrisch verbindbar ist, wenn der Gegenstand (10) im Aufnahmeraum (210i) aufgenommen ist, und
wobei der zweite Prozessor (230) konfiguriert ist zum: Empfangen von Identifikationsinformationen aus dem Speicher (11) des Gegenstands (10) über die mindestens eine elektrische Verbindungseinheit (260), wenn der Gegenstand (10) im Aufnahmeraum (210i) aufgenommen ist; und auf der Grundlage der empfangenen Identifikationsinformationen, Detektieren eines Typs des Gegenstands (10), der im Aufnahmeraum (210i) aufgenommen ist.

7. Gehirnstimulationssystem nach Anspruch 6, das ferner eine Verbindungseinrichtung (300) umfasst, die ein Ende, das mit der Gehirnstimulationsvorrichtung (100) verbunden ist, und ein weiteres Ende, das mit der Steuervorrichtung (200) verbunden ist, aufweist, wobei der erste Prozessor (130) konfiguriert ist, vom zweiten Prozessor (230) über die Verbindungseinrichtung (300) ein Signal zu empfangen, das Daten enthält, die dem detektierten Typ des Gegenstands (10) entsprechen.

8. Gehirnstimulationssystem nach Anspruch 6, wobei die Gehirnstimulationsvorrichtung (100) ferner eine erste Kommunikationseinrichtung enthält, die Steuervorrichtung (200) ferner eine zweite Kommunikationseinrichtung zur Kommunikation mit der ersten Kommunikationseinrichtung enthält und der erste Prozessor (130) konfiguriert ist, vom zweiten Prozessor (230) über die erste Kommunikationseinrichtung und die zweite Kommunikationseinrichtung ein Signal zu empfangen, das Daten enthält, die dem detektierten Typ des Gegenstands (10) entsprechen.

9. Gehirnstimulationssystem nach Anspruch 6, wobei die Steuervorrichtung (200) ferner mindestens eine Knopfeinheit (220) enthält, wobei der zweite Prozessor (230) konfiguriert ist, an den ersten Prozessor (130) ein Signal zum Steuern der Leistung der Gehirnstimulationsvorrichtung (100) oder einer Intensität eines Stroms, der von der Ausgangseinheit (150) an den Körper (B) des Benutzers angelegt wird, auf der Grundlage einer Benutzereingabe in die mindestens eine Knopfeinheit (220) zu senden.

10. Gehirnstimulationssystem nach Anspruch 6, wobei der zweite Prozessor (230) konfiguriert ist, an den ersten Prozessor (130) ein erstes Signal zu senden, das Daten enthält, die angeben, dass ein erster Gegenstand (10) aufgenommen ist, wenn detektiert wird, dass der erste Gegenstand (10) im Aufnahmeraum (210i) aufgenommen ist, und der erste Prozessor (130) konfiguriert ist, auf der Grundlage des ersten Signals, das vom zweiten Prozessor (230) empfangen wird, über die Ausgangseinheit (150) einen Strom, der einem ersten Stromprofil entspricht, in den Körper (B) des Benutzers einzugeben, derart, dass eine Gehirnstimulation, die einem ersten Gehirnstimulationsprofil entspricht, auf den Benutzer angewendet wird.

11. Gehirnstimulationssystem nach Anspruch 10, wobei der zweite Prozessor (230) konfiguriert ist, an den ersten Prozessor (130) ein zweites Signal zu senden, das Daten enthält, die angeben, dass ein zweiter Gegenstand aufgenommen ist, wenn detektiert wird, dass der zweite Gegenstand, der vom ersten Gegenstand (10) verschieden ist, im Aufnahmeraum (210i) aufgenommen ist, und der erste Prozessor (130) konfiguriert ist, auf der Grundlage des zweiten Signals, das vom zweiten Prozessor (230) empfangen wird, über die Ausgangseinheit (150) einen Strom, der einem zweiten Stromprofil entspricht, in den Körper (B) des Benutzers einzugeben, derart, dass eine Gehirnstimulation, die einem zweiten Gehirnstimulationsprofil entspricht, auf den Benutzer angewendet wird.

## Revendications

1. Dispositif de stimulation cérébrale (100) comportant :
un boîtier (110) ayant au moins une zone configurée pour être fixée sur un corps (B) d'un utilisateur, et incluant un espace de réception (110i) destinée à recevoir un article comportant une mémoire (11) ;
une batterie (140) agencée à l'intérieur du boîtier (110) ;
une unité de sortie (150) configurée pour appliquer un courant au corps (B) de l'utilisateur sur la base de l'énergie fournie à partir de la batterie (140) ; et
un processeur (130) électriquement connecté à la batterie (140) et à l'unité de sortie (150), et configuré pour : détecter un type de l'article (10) reçu dans l'espace de réception (110i) ; et sur la base du type détecté de l'article (10), appliquer un courant au corps (B) de l'utilisateur via l'unité de sortie (150) de sorte qu'une stimulation cérébrale est appliquée à l'utilisateur,
dans lequel le dispositif de stimulation cérébrale (100) comporte en outre au moins une unité de connexion électrique (260) agencée dans l'espace de réception (110), dans lequel la au moins une unité de connexion électrique (260) peut être électriquement connectée à la mémoire (11) de l'article (10) lorsque l'article (10) est reçu dans l'espace de réception (110i), et
dans lequel le processeur (130) est configuré pour : recevoir des informations d'identification provenant de la mémoire (11) de l'article (10) via la au moins une unité de connexion électrique (260) lorsque l'article (10) est reçu dans l'espace de réception (110i) ; et sur la base des informations d'identification reçues, détecter le type de l'article (10) reçu dans l'espace de réception (110i).

2. Dispositif de stimulation cérébrale selon la revendication 1, comportant en outre au moins une unité de bouton (220) configurée pour recevoir une entrée de l'utilisateur, dans lequel le processeur (130) est configuré pour commander l'énergie du dispositif de stimulation cérébrale (100) ou une intensité d'un courant appliqué au corps (B) de l'utilisateur, sur la base d'une entrée d'utilisateur dans la au moins une unité de bouton (220).

3. Dispositif de stimulation cérébrale selon la revendication 1, dans lequel le processeur (130) est configuré pour : lors de la détection qu'un premier article est reçu dans l'espace de réception (110i), appliquer un courant correspondant à un premier profil de courant au corps (B) de l'utilisateur via l'unité de sortie (150) de sorte qu'une stimulation cérébrale correspondant à un premier profil de stimulation cérébrale est appliquée à l'utilisateur ; et lors de la détection qu'un second article différent du premier article est reçu dans l'espace de réception (110i), appliquer un courant correspondant à un second profil de courant au corps de l'utilisateur via l'unité de sortie (150) de sorte qu'une stimulation cérébrale correspondant à un second profil de stimulation cérébrale est appliquée à l'utilisateur.

4. Dispositif de stimulation cérébrale selon la revendication 1, dans lequel le processeur (130) est configuré pour : compter un nombre de fois où un courant est appliqué au corps de l'utilisateur via l'unité de sortie (150) ; et arrêter un fonctionnement de l'unité de sortie (150) lorsque le nombre de fois compté est supérieur à une valeur prédéfinie.

5. Dispositif de stimulation cérébrale selon la revendication 1, comportant en outre un élément de communication configuré pour transmettre un signal à un dispositif externe (20) ou recevoir un signal provenant de celui-ci, dans lequel le processeur (130) est configuré pour commander un fonctionnement du dispositif de stimulation cérébrale (100), sur la base d'un signal reçu du dispositif externe (20) via l'élément de communication.

6. Système de stimulation cérébrale (1000) comportant :
un dispositif de stimulation cérébrale (100) ayant au moins une zone pouvant être fixée sur le corps (B) d'un utilisateur ; et
un dispositif de commande (200) connecté au dispositif de stimulation cérébrale (100) de manière opérationnelle,
dans lequel le dispositif de stimulation cérébrale (100) inclut : un premier boîtier (110) ayant la au moins une zone pouvant être fixée sur le corps (B) de l'utilisateur ; une batterie (140) agencée à l'intérieur du premier boîtier (110) ; une unité de sortie (150) configurée pour appliquer un courant au corps (B) de l'utilisateur, sur la base de l'énergie fournie à partir de la batterie (140) ; et un premier processeur (130) électriquement connecté à la batterie (140) et à l'unité de sortie (150), et
le dispositif de commande (200) inclut : un second boîtier (210) incluant un espace de réception (210i) destiné à recevoir un article (10) comportant une mémoire (11) ; et un second processeur (230) configuré pour détecter un type de l'article (10) reçu dans l'espace de réception (210i),
dans lequel le premier processeur (130) est configuré pour : recevoir, à partir du second processeur (230), un signal incluant des données correspondant au type détecté de l'article (10) ; et sur la base du signal reçu, appliquer un courant au corps (B) de l'utilisateur via l'unité de sortie (150) de sorte qu'une stimulation cérébrale est appliquée à l'utilisateur,
dans lequel le dispositif de commande (200) inclut en outre au moins une unité de connexion électrique (260) agencée dans l'espace de réception (210i), dans lequel la au moins une unité de connexion électrique (260) peut être électriquement connectée à la mémoire (11) de l'article (10) lorsque l'article (10) est reçu dans l'espace de réception (210i), et
dans lequel le second processeur (230) est configuré pour : recevoir des informations d'identification provenant de la mémoire (11) de l'article (10) via la au moins une unité de connexion électrique (260) lorsque l'article (10) est reçu dans l'espace de réception (210i) ; et sur la base des informations d'identification reçues, détecter le type de l'article (10) reçu dans l'espace de réception (210i).

7. Système de stimulation cérébrale selon la revendication 6, comportant en outre un connecteur (300) ayant une extrémité connectée au dispositif de stimulation cérébrale (100) et une autre extrémité connectée au dispositif de commande (200), dans lequel le premier processeur (130) est configuré pour recevoir, à partir du second processeur (230) via le connecteur (300), un signal incluant des données correspondant au type détecté de l'article (10).

8. Système de stimulation cérébrale selon la revendication 6, dans lequel le dispositif de stimulation cérébrale (100) inclut en outre un premier élément de communication, le dispositif de commande (200) inclut en outre un second élément de communication pour une communication avec le premier élément de communication, et le premier processeur (130) est configuré pour recevoir, à partir du second processeur (230) via le premier élément de communication et le second élément de communication, un signal incluant des données correspondant au type détecté de l'article (10).

9. Système de stimulation cérébrale selon la revendication 6, dans lequel le dispositif de commande (200) inclut en outre au moins une unité de bouton (220), dans lequel le second processeur (230) est configuré pour transmettre, au premier processeur (130), un signal destiné à commander l'énergie du dispositif de stimulation cérébrale (100) ou une intensité d'un courant appliqué à partir de l'unité de sortie (150) au corps (B) de l'utilisateur, sur la base d'une entrée d'utilisateur dans la au moins une unité de bouton (220).

10. Système de stimulation cérébrale selon la revendication 6, dans lequel le second processeur (230) est configuré pour transmettre, au premier processeur (130), un premier signal incluant des données indiquant qu'un premier article (10) est reçu, lors de la détection que le premier article (10) est reçu dans l'espace de réception (210i), et le premier processeur (130) est configuré pour appliquer un courant correspondant à un premier profil de courant au corps (B) de l'utilisateur via l'unité de sortie (150) de sorte qu'une stimulation cérébrale correspondant à un premier profil de stimulation cérébrale est appliquée à l'utilisateur, sur la base du premier signal reçu du second processeur (230).

11. Système de stimulation cérébrale selon la revendication 10, dans lequel le second processeur (230) est configuré pour transmettre, au premier processeur (130), un second signal incluant des données indiquant qu'un second article est reçu, lors de la détection que le second article différent du premier article (10) est reçu dans l'espace de réception (210i), et le premier processeur (130) est configuré pour appliquer un courant correspondant à un second profil de courant au corps (B) de l'utilisateur via l'unité de sortie (150) de sorte qu'une stimulation cérébrale correspondant à un second profil de stimulation cérébrale est appliquée à l'utilisateur, sur la base du second signal reçu du second processeur (230).
